# FASCICULE DE BREVET EUROPEEN

(11) **EP 3 634 620 B1**
(45) Date de publication et mention de la délivrance du brevet: **08.11.2023**
(21) Numéro de dépôt: 18727832.0
(22) Date de dépôt: 05.06.2018
(51) Int. Cl.: B01J 19/02, B23K 20/00, C07C 19/08, C07C 21/18

(54) **PROCEDE DE MODIFICATION DE LA DISTRIBUTION EN FLUOR DANS UN COMPOSE HYDROCARBURE**
VERFAHREN ZUR MODIFIZIERUNG DER FLUORVERTEILUNG IN EINER KOHLENWASSERSTOFFVERBINDUNG
METHOD FOR MODIFYING FLUORINE DISTRIBUTION IN A HYDROCARBON COMPOUND

(30) Priorité: 06.06.2017 FR 1754981
(43) Date de publication de la demande: 15.04.2020
(73) Titulaire: Arkema France, 92700 Colombes (FR)
(72) Inventeur: DEUR-BERT, Dominique, 69390 Charly (FR); WENDLINGER, Laurent, 69510 Soucieu en Jarrest (FR)
(74) Mandataire: Arkema Patent
(86) Numéro de dépôt international: PCT/EP2018/064713
(87) Numéro de publication internationale: WO 2018/224476

(56) Documents cités:
- WO-A1-94/06554
- WO-A1-2009/118630
- US-A1- 2005 019 487
- US-A1- 2009 240 090
- US-A1- 2014 066 670

## Description

### Domaine technique

La présente invention se rapporte à des procédés de modification de la distribution en fluor en phase gazeuse. En particulier, la présente invention se rapporte à des procédés de modification de la distribution en fluor en phase gazeuse en présence d'un catalyseur à base de chrome.

### Arrière-plan technologique de l'invention

Les hydrocarbures halogénés, en particulier les hydrocarbures fluorés comme les hydrofluorooléfines, sont des composés qui ont une structure utile comme matériaux fonctionnels, solvants, réfrigérants, agents de gonflage et monomères pour polymères fonctionnels ou matériaux de départ pour de tels monomères. Des hydrofluorooléfines comme le 2,3,3,3-tétrafluoropropène (HFO-1234yf) attirent l'attention parce qu'elles offrent un comportement prometteur comme réfrigérants à faible potentiel de réchauffement global.

Les procédés de production de fluorooléfines sont habituellement effectués en présence d'une substance de départ telle qu'un alcane contenant du chlore ou un alcène contenant du chlore, et en présence d'un agent fluorant tel que le fluorure d'hydrogène. Ces procédés peuvent être effectués en phase gazeuse ou en phase liquide, en absence ou non de catalyseur.

Les procédés en phase gazeuse sont habituellement effectués en présence de catalyseurs et d'acide fluorhydrique. L'environnement à l'intérieur du réacteur catalytique présente une très forte acidité engendrant une corrosion élevée du matériau du réacteur. Les réacteurs utilisés dans les procédés impliquant de l'acide fluorhydrique comprennent généralement un matériau de base et un matériau résistant à la corrosion. Le matériau de base et le matériau résistant à la corrosion peuvent être assemblés par différentes techniques dans lesquelles les matériaux sont fondus ou non. Suivant la technique d'assemblage utilisée, les propriétés des matériaux peuvent être différentes.

Par exemple, lorsque les matériaux sont fondus, des faiblesses peuvent apparaître à l'interface de ceux-ci au cours du temps en présence d'un environnement acide.

L'assemblage de matériaux par placage (sans fonte des matériaux) est une technique peu onéreuse. Cependant, on connaît par US 5,565,393 un procédé de fluoration effectué dans un réacteur dont les matériaux sont assemblés par placage en présence de catalyseur de type tantale, niobium ou antimoine. Les matériaux se corrodant le moins sont des alliages de type molybdène/rhénium ou tungstène/rhénium ou des alliages à base d'or. Le coût de ce type d'alliage est trop important pour être applicable à l'échelle industrielle. En outre, le taux de corrosion des matériaux utilisés est supérieur à 10 mm/an. Le taux de corrosion est lui aussi beaucoup trop élevé pour permettre l'utilisation de réacteur préparé par cette technique. Les réacteurs dont les matériaux sont assemblés par placage ne sont ainsi pas compatibles avec des procédés de fluoration dans les conditions décrites par US 5,565,393.

WO 2009/118630 divulgue un procédé de déhydrofluoration mis en oeuvre dans un réacteur comprenant un revêtement intérieur en alliage de nickel avec plus de 40% en poids. US 2005/019487 divulgue un procédé de fluoration dans un réacteur obtenu par placage avec une couche intérieure comprenant plus de 40% de nickel en poids.

Il existe donc un besoin pour des procédés de fluoration effectués dans des conditions minimisant la corrosion et augmentant la durée de vie d'un catalyseur.

### Résumé de l'invention

De manière surprenante, le demandeur a constaté qu'en présence d'un catalyseur à base de chrome, la corrosion des matériaux utilisés dans un réacteur de fluoration lorsque ceux-ci sont assemblés par placage étant fortement diminuée permettant la mise en oeuvre d'un procédé fiable et économiquement viable à l'échelle industrielle.

Selon un premier aspect, l'invention fournit un procédé de modification de la distribution en fluor dans un composé hydrocarbure, comprenant une étape de mise en contact entre ledit composé hydrocarbure et une composition catalytique comprenant un catalyseur à base de chrome, ledit procédé étant mis en oeuvre dans un réacteur fait d'un matériau comprenant une couche de base faite d'un matériau **M1** et une couche intérieure faite d'un matériau **M2**, ladite couche de base et ladite couche intérieure étant disposées l'une contre l'autre par placage.

Selon un mode de réalisation préféré, le taux de corrosion du matériau **M2**, mesuré suivant ASTM D 2 328-65 T, est inférieur à 1 mm/an.

Selon l'invention le placage est réalisé par placage par soudure, placage par explosion, placage par laminage à chaud ou placage par laminage à froid, de préférence par placage par explosion ou placage par laminage à chaud.

Selon un mode de réalisation préféré, le matériau **M2** est en contact avec le composé hydrocarbure et a une résistance à la traction inférieure à celle du matériau **M1.**

Selon un mode de réalisation préféré, le matériau **M2** est en contact avec le composé hydrocarbure et a une élongation supérieure à celle du matériau **M1.**

Selon un mode de réalisation préféré, ladite couche intérieure a une épaisseur comprise entre 0,05 et 10 mm, ladite épaisseur de ladite couche intérieure étant inférieure à celle de ladite couche de base.

Selon l'invention, le matériau **M2** comprend au moins 40% en poids de nickel sur base du poids total du matériau **M2.**

Selon l'invention, le matériau **M1** comprend au moins 70% de fer, avantageusement ladite couche de base comprend moins de 0,2% de carbone et/ou moins de 1% de molybdène et/ou moins de 2% de chrome sur base du poids total du matériau **M1.**

Selon un mode de réalisation préféré, ledit composé hydrocarbure est de formule (I) CX(Y)₂-CX(Y)ₘ-CHₘXY où X et Y représentent indépendamment H, F ou CI et m = 0 ou 1 avec au moins un parmi X ou Y qui est CI ou F.

Selon un mode de réalisation préféré, le composé hydrocarbure est choisi dans le groupe constitué du tétrachloropropène, chlorotrifluoropropène, pentachloropropane, dichlorotrifluoropropane, trichlorodifluoropropane, tétrafluorochloropropane, tétrachlorofluoropropane, dichlorodifluoropropène, trichlorofluoropropène, pentafluoropropane et leurs mélanges ; de préférence, le composé hydrocarbure est choisi dans le groupe constitué du 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

Selon un mode de réalisation préféré, la teneur en fluor du composé hydrocarbure est augmentée en faisant réagir ledit composé hydrocarbure avec du fluorure d'hydrogène en phase gazeuse en présence de ladite composition catalytique, le composé hydrocarbure étant un hydrocarbure saturé halogéné ou un hydrocarbure insaturé halogéné ou un hydrocarbure insaturé.

Selon un mode de réalisation préféré, la teneur en fluor du composé hydrocarbure est diminuée par déshydrofluoration dudit composé hydrocarbure en présence de ladite composition catalytique, ledit composé hydrocarbure étant un composé hydrocarbure fluoré.

Selon un mode de réalisation préféré, la distribution en fluor du composé hydrocarbure est modifiée en isomérisant ledit composé hydrocarbure en présence de ladite composition catalytique, ledit composé hydrocarbure étant un composé hydrocarbure fluoré.

Selon un mode de réalisation préféré, la distribution en fluor du composé hydrocarbure est modifiée en dismutant ledit composé hydrocarbure en phase gazeuse en présence de ladite composition catalytique, ledit composé hydrocarbure étant un composé hydrocarbure chlorofluoré.

Selon un mode de réalisation préféré, la teneur en fluor du composé hydrocarbure est diminuée en faisant réagir ledit composé hydrocarbure avec du chlorure d'hydrogène en phase gazeuse en présence de ladite composition catalytique, ledit composé hydrocarbure étant un composé hydrocarbure halogéné contenant au moins un atome de fluor.

Selon un mode de réalisation préféré, la teneur en fluor d'un premier composé hydrocarbure est augmentée en faisant réagir ledit premier composé hydrocarbure avec du fluorure d'hydrogène en phase gazeuse en présence d'une composition catalytique comprenant un catalyseur à base de chrome, le premier composé hydrocarbure étant un hydrocarbure saturé halogéné ou un hydrocarbure insaturé halogéné ou un hydrocarbure insaturé, et dans lequel la teneur en fluor d'un second composé hydrocarbure est diminuée en déshydrofluorant ledit second composé hydrocarbure en présence de ladite composition catalytique, ledit second composé hydrocarbure étant un composé hydrocarbure fluoré.

### Description détaillée de l'invention

Dans un premier aspect de la présente invention, un procédé de modification de la distribution en fluor dans un composé hydrocarbure en présence d'une composition catalytique est fourni. Dans ce procédé, la composition catalytique comprend un catalyseur à base de chrome. De préférence, le catalyseur à base de chrome peut être un oxyde de chrome (par exemple CrO₃ ou Cr₂O₃), un oxyfluorure de chrome ou un fluorure de chrome (par exemple CrF₃) ou un mélange de ceux-ci. L'oxyfluorure de chrome peut contenir une teneur en fluor comprise entre 1 et 60% en poids sur base du poids total de l'oxyfluorure de chrome, avantageusement entre 5 et 55% en poids, de préférence entre 10 et 52% en poids, plus préférentiellement entre 15 et 52% en poids, en particulier entre 20 et 50% en poids, plus particulièrement entre 25 et 45% en poids, de manière privilégiée entre 30 et 45% en poids, de manière plus privilégiée de 35 à 45% en poids de fluor sur base du poids total de l'oxyfluorure de chrome. La composition catalytique peut également comprendre un co-catalyseur choisi parmi le groupe consistant en Ni, Co, Zn, Mg, Mn, Fe, Zn, Ti, V, Zr, Mo, Ge, Sn, Pb, Sb ; de préférence Ni, Co, Zn, Mg, Mn ; en particulier Ni, Co, Zn. La teneur en poids du co-catalyseur est comprise entre 1 et 10% en poids sur base du poids total de la composition catalytique. La composition catalytique peut également comprendre un support tel que l'alumine, par exemple sous sa forme alpha, de l'alumine activée, les halogénures d'aluminium (AlF₃ par exemple), les oxyhalogénures d'aluminium, du charbon actif, fluorure de magnésium ou du graphite. De préférence, la composition catalytique a une surface spécifique entre 1 et 100 m²/g, de préférence entre 5 et 80 m²/g, plus préférentiellement entre 5 et 70 m²/g, idéalement entre 5 et 50 m²/g, en particulier entre 10 et 50 m²/g, plus particulièrement entre 15 et 45 m²/g.

On peut utiliser les catalyseurs fournis selon la présente invention pour modifier la distribution en fluor dans des composés hydrocarbures, ces derniers étant des composés hydrocarbures halogénés ou non. On peut modifier la distribution en fluor dans un composé hydrocarbure en augmentant la teneur en fluor du composé hydrocarbure. On peut aussi modifier la distribution en fluor d'un composé hydrocarbure en diminuant la teneur en fluor du composé hydrocarbure et/ou en réarrangeant la position d'atomes de fluor sur les atomes de carbone du composé hydrocarbure.

La présente invention peut fournir des procédés où on modifie la distribution en fluor dans des composés hydrocarbures contenant entre un et douze atomes de carbone, de préférence des procédés où on modifie la distribution en fluor dans des composés hydrocarbures contenant entre un et six atomes de carbone, en particulier des procédés où on modifie la distribution en fluor dans des composés hydrocarbures contenant trois atomes de carbone, plus particulièrement où on modifie la distribution en fluor dans des composés hydrocarbures halogénés contenant trois atomes de carbone. La présente invention peut fournir des procédés où on augmente la teneur en fluor de composés hydrocarbures contenant entre un et douze atomes de carbone, de préférence des procédés où on augmente la teneur en fluor de composés hydrocarbures contenant entre un et six atomes de carbone, en particulier des procédés où on augmente la teneur en fluor de composés hydrocarbures contenant trois atomes de carbone, plus particulièrement des procédés où on augmente la teneur en fluor de composés hydrocarbures halogénés contenant trois atomes de carbone. Les procédés de modification de la distribution en fluor de composés hydrocarbures, de préférence de composés hydrocarbures halogénés, incluent la fluoration, la chlorofluoration, l'isomérisation, dismutation, déshydrofluoration et chlorodéfluoration.

Les composés hydrocarbures incluent ceux de formule générale CₕHₐBr_{b}Cl_{c}F_{d}, où h est un entier entre 1 et 6, a est un entier entre 0 et 13, b est un entier entre 0 et 4, c est un entier entre 0 et 13, d est un entier entre 0 et 13, et la somme de a, b, c et d est égale à 2h+2 ; ou ceux de formule générale CₚHₑBr_{f}Cl_{g}Fₕ, où p est un entier entre 2 et 6, e est un entier entre 0 et 10, f est un entier entre 0 et 2, g est un entier entre 0 et 12, h est un entier entre 0 et 11, et la somme de e, f, g et h est égale à 2p. De préférence, les composés hydrocarbures incluent ceux de formule générale CₕHₐCl_{c}F_{d}, où h est un entier entre 2 et 4, a est un entier entre 0 et 9, c est un entier entre 0 et 9, d est un entier entre 0 et 9, et la somme de a, c et d est égal à 2h+2 ; ou ceux de formule générale CₚHₑCl_{g}Fₕ, où p est un entier entre 2 et 4, e est un entier entre 0 et 8, g est un entier entre 0 et 8, h est un entier entre 0 et 7, et la somme de e, f, g et h est égal à 2p.

En particulier, les composés hydrocarbures qui conviennent aux procédés selon la présente invention sont de formule (I) CX(Y)₂-CX(Y)ₘ-CHₘXY, où X et Y représentent indépendamment H, F ou CI et m = 0 ou 1 avec au moins un parmi X ou Y étant CI ou F. De préférence, les composés hydrocarbures peuvent être choisis dans le groupe constitué de tétrachloropropène, chlorotrifluoropropène, pentachloropropane, dichlorotrifluoropropane, trichlorodifluoropropane, tétrachlorofluoropropane, dichlorodifluoropropène, trichlorofluoropropène, pentafluoropropane, tétrafluorochloropropane et leurs mélanges.

De préférence, les composés hydrocarbures peuvent être choisis dans le groupe constitué de 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

Ledit procédé selon la présente invention est mis en oeuvre dans un réacteur fait d'un matériau comprenant une couche de base faite d'un matériau **M1** et une couche intérieure faite d'un matériau **M2**, ladite couche de base et ladite couche intérieure étant disposées l'une contre l'autre par placage. Le réacteur peut être un réacteur à lit catalytique fixe ou un réacteur à lit catalytique fluidisé ou un réacteur multitubulaire. De préférence, ledit réacteur est un réacteur à lit catalytique fixe.

Selon un mode de réalisation préféré, le taux de corrosion du matériau **M2** est inférieur à 1 mm/an, avantageusement inférieur à 0,5 mm/an, de préférence inférieur à 0,1 mm/an, plus préférentiellement inférieur à 0,05 mm/an, en particulier inférieur à 0,025 mm/an, plus particulièrement inférieur 10 µm/an, de manière privilégiée inférieur à 5 µm/an. Ce taux est mesuré selon la méthode des coupons ASTM D 2 328-65 T.

Selon l'invention, le placage est réalisé par placage par soudure, placage par explosion, placage par laminage à chaud ou placage par laminage à froid. De préférence, le placage est réalisé par explosion ou par laminage à chaud. En particulier, le placage est réalisé par explosion.

Le matériau **M2** est en contact avec le composé hydrocarbure. De préférence, le matériau **M2** peut avoir une résistance à la traction inférieure à celle du matériau **M1.** De préférence, le matériau **M2** peut avoir une élongation supérieure à celle du matériau **M1.** En particulier, le matériau **M2** peut avoir une résistance à la traction inférieure à celle du matériau **M1** et une élongation supérieure à celle du matériau **M1.**

Selon un mode de réalisation préféré, ladite couche intérieure a une épaisseur comprise entre 0,01 et 20 mm, ladite épaisseur de ladite couche intérieure étant inférieure à celle de ladite couche de base. De préférence, ladite couche intérieure peut avoir une épaisseur comprise entre 0,05 et 15 mm, de préférence entre 0,1 et 10 mm, plus préférentiellement entre 0,1 et 5 mm.

Le matériau **M2** comprend au moins 40% en poids de nickel sur base du poids total du matériau **M2**. De préférence, le matériau **M2** comprend au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**.

Le matériau **M2** peut également comprendre du chrome dans une teneur inférieure à 35% en poids sur base du poids total du matériau **M2**, avantageusement inférieure à 30% en poids, de préférence inférieure à 20% en poids, plus préférentiellement inférieure à 15% en poids, en particulier inférieure à 10% en poids, plus particulièrement inférieure à 5% en poids sur base du poids total du matériau **M2.**

Le matériau **M2** peut également comprendre du molybdène dans une teneur inférieure à 35% en poids sur base du poids total du matériau **M2**, avantageusement inférieure à 30% en poids, de préférence inférieure à 20% en poids, plus préférentiellement inférieure à 15% en poids, en particulier inférieure à 10% en poids, plus particulièrement inférieure à 5% en poids sur base du poids total du matériau **M2.**

De préférence, le matériau **M2** comprend au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**; et moins de 35% en poids de chrome, avantageusement moins de 30% en poids, de préférence moins de 20% en poids, plus préférentiellement moins de 15% en poids, en particulier moins de 10% en poids, plus particulièrement moins de 5% en poids de chrome sur base du poids total du matériau **M2**; et moins de 35% en poids de molybdène, avantageusement moins de 30% en poids, de préférence moins de 20% en poids, plus préférentiellement moins de 15% en poids, en particulier moins de 10% en poids, plus particulièrement moins de 5% en poids de molybdène, sur base du poids total du matériau **M2.**

Le matériau **M2** peut également comprendre du cobalt dans une teneur inférieure à 10% en poids sur base du poids total du matériau **M2,** avantageusement inférieure à 8% en poids, de préférence inférieure à 6% en poids, plus préférentiellement inférieure à 4% en poids, en particulier inférieure à 3% en poids, plus particulièrement inférieure à 2% en poids sur base du poids total du matériau **M2.**

Le matériau **M2** peut également comprendre du tungstène dans une teneur inférieure à 10% en poids sur base du poids total du matériau **M2**, avantageusement inférieure à 9% en poids, de préférence inférieure à 8% en poids, plus préférentiellement inférieure à 7% en poids, en particulier inférieure à 6% en poids, plus particulièrement inférieure à 5% en poids sur base du poids total du matériau **M2.**

Le matériau **M2** peut également comprendre du fer dans une teneur inférieure à 25% en poids sur base du poids total du matériau **M2**, avantageusement inférieure à 20% en poids, de préférence inférieure à 15% en poids, plus préférentiellement inférieure à 10% en poids, en particulier inférieure à 7% en poids, plus particulièrement inférieure à 5% en poids sur base du poids total du matériau **M2.**

Le matériau **M2** peut également comprendre du manganèse dans une teneur inférieure à 5% en poids sur base du poids total de l'alliage, avantageusement inférieure à 4% en poids, de préférence inférieure à 3% en poids, plus préférentiellement inférieure à 2% en poids, en particulier inférieure à 1% en poids, plus particulièrement inférieure à 0,5% en poids sur base du poids total du matériau **M2.**

Le matériau **M2** peut également comprendre du cuivre dans une teneur inférieure à 50% en poids, avantageusement inférieure à 45% en poids, de préférence inférieure à 40% en poids, plus préférentiellement inférieure à 35% en poids, en particulier inférieure à 30% en poids, plus particulièrement inférieure à 25% en poids de cuivre sur base du poids total du matériau **M2**.

Le matériau **M2** comprend au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**; et moins de 50% en poids, avantageusement inférieure à 45% en poids, de préférence inférieure à 40% en poids, plus préférentiellement inférieure à 35% en poids, en particulier inférieure à 30% en poids, plus particulièrement inférieure à 25% en poids de cuivre sur base du poids total du matériau **M2.**

Le matériau **M2** comprend au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**; et moins de 35% en poids de chrome, avantageusement moins de 30% en poids, de préférence moins de 20% en poids, plus préférentiellement moins de 15% en poids, en particulier moins de 10% en poids, plus particulièrement moins de 5% en poids de chrome sur base du poids total du matériau **M2**; et moins de 35% en poids de molybdène, avantageusement moins de 30% en poids, de préférence moins de 20% en poids, plus préférentiellement moins de 15% en poids, en particulier moins de 10% en poids, plus particulièrement moins de 5% en poids de molybdène sur base du poids total du matériau **M2**; et moins de 25% en poids de fer, avantageusement moins de 20% en poids, de préférence moins de 15% en poids, plus préférentiellement moins de 10% en poids, en particulier moins de 7% en poids, plus particulièrement moins de 5% en poids de fer sur base du poids total du matériau **M2.**

Le matériau **M2** peut comprendre moins de 4% en poids de titane sur base du poids total du matériau **M2**, avantageusement moins de 3% en poids, de préférence moins de 2% en poids, plus préférentiellement moins de 1% en poids, en particulier moins de 0,5% en poids de titane, plus particulièrement moins de 0,05% en poids de titane sur base du poids total du matériau **M2**, de manière privilégiée le matériau **M2** est dépourvu de titane.

Le matériau **M2** peut comprendre moins de 4% en poids de niobium sur base du poids total du matériau **M2**, avantageusement moins de 3% en poids, de préférence moins de 2% en poids, plus préférentiellement moins de 1% en poids, en particulier moins de 0,5% en poids de titane, plus particulièrement moins de 0,05% en poids de niobium sur base du poids total du matériau **M2**, de manière privilégiée le matériau **M2** est dépourvu de niobium.

Le matériau **M1** comprend au moins 70% en poids de fer, avantageusement au moins 75% en poids, de préférence au moins 80% en poids, plus préférentiellement au moins 85% en poids, en particulier au moins 90% en poids, plus particulièrement au moins 95% en poids de fer sur base du poids total du matériau **M1.**

Le matériau **M1** peut également comprendre moins de 2% en poids de carbone, avantageusement moins de 1,5% en poids, de préférence moins de 1% en poids, plus préférentiellement moins de 0,75% en poids, en particulier moins de 0,5% en poids, plus particulièrement moins de 0,2% en poids, de manière privilégiée moins de 0,1% en poids sur base du poids total du matériau **M1.**Plus particulièrement, le matériau **M1** peut comprendre entre 0,01 et 0,2% en poids de carbone sur base du poids total du matériau **M1.**

Le matériau **M1** peut également comprendre moins de 2% en poids de molybdène, avantageusement moins de 1,5% en poids, de préférence moins de 1,25% en poids, plus préférentiellement moins de 1% en poids de molybdène sur base du poids total du matériau **M1.**Plus particulièrement, le matériau **M1** peut comprendre entre 0,1 et 1% en poids de molybdène sur base du poids total du matériau **M1.**

Le matériau **M1** peut également comprendre moins de 5% en poids de chrome, avantageusement moins de 4% en poids, de préférence moins de 3% en poids, plus préférentiellement moins de 2% en poids, en particulier moins de 1% en poids de chrome sur base du poids total du matériau **M1.**Plus particulièrement, le matériau **M1** peut comprendre entre 0,5 et 2% en poids de chrome sur base du poids total du matériau **M1.**

Le matériau **M1** peut également comprendre moins de 2% en poids de silicium, avantageusement moins de 1,5% en poids, de préférence moins de 1,25% en poids, plus préférentiellement moins de 1% en poids de silicium sur base du poids total du matériau **M1.** Plus particulièrement, le matériau **M1** peut comprendre entre 0,1 et 1,5 % en poids de silicium sur base du poids total du matériau **M1.**

Le matériau **M1** peut également comprendre moins de 2% en poids de manganèse, avantageusement moins de 1,5% en poids, de préférence moins de 1,25% en poids, plus préférentiellement moins de 1% en poids de manganèse sur base du poids total du matériau **M1.**Plus particulièrement, le matériau **M1** peut comprendre entre 0,1 et 1% en poids de manganèse sur base du poids total du matériau **M1.**

Ainsi le réacteur utilisé dans les procédés selon l'invention comprend une couche de base faite d'un matériau **M1** et une couche intérieure, en contact avec au moins le composé hydrocarbure, faite d'un matériau **M2** disposées l'une contre l'autre par placage ; ledit matériau **M2** comprenant :
- au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**; et moins de 35% en poids de chrome, avantageusement moins de 30% en poids, de préférence moins de 20% en poids, plus préférentiellement moins de 15% en poids, en particulier moins de 10% en poids, plus particulièrement moins de 5% en poids de chrome sur base du poids total du matériau **M2**; et moins de 35% en poids de molybdène, avantageusement moins de 30% en poids, de préférence moins de 20% en poids, plus préférentiellement moins de 15% en poids, en particulier moins de 10% en poids, plus particulièrement moins de 5% en poids de molybdène sur base du poids total du matériau **M2**; et moins de 25% en poids de fer avantageusement moins de 20% en poids, de préférence moins de 15% en poids, plus préférentiellement moins de 10% en poids, en particulier moins de 7% en poids, plus particulièrement moins de 5% en poids de fer sur base du poids total du matériau **M2** ; ou
- au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2**; et moins de 50% en poids, avantageusement inférieure à 45% en poids, de préférence inférieure à 40% en poids, plus préférentiellement inférieure à 35% en poids, en particulier inférieure à 30% en poids, plus particulièrement inférieure à 25% en poids de cuivre sur base du poids total du matériau **M2** ; ou
- au moins 40% en poids de nickel sur base du poids total du matériau **M2**, de préférence au moins 45 % en poids de nickel, plus préférentiellement au moins 50% en poids de nickel, en particulier au moins 55% en poids de nickel, plus particulièrement au moins 60% en poids de nickel, de manière privilégiée au moins 65% en poids de nickel, de manière plus privilégiée au moins 70% en poids de nickel sur base du poids total du matériau **M2** ; et moins de 35% en poids de chrome, avantageusement moins de 30% en poids, de préférence moins de 20% en poids, plus préférentiellement moins de 15% en poids, en particulier moins de 10% en poids, plus particulièrement moins de 5% en poids de chrome sur base du poids total du matériau **M2** ; et moins de 35% en poids de molybdène, avantageusement moins de 30% en poids, de préférence moins de 20% en poids, plus préférentiellement moins de 15% en poids, en particulier moins de 10% en poids, plus particulièrement moins de 5% en poids de molybdène sur base du poids total du matériau **M2** ;
et le matériau **M1** comprenant :
- au moins 70% en poids de fer, avantageusement au moins 75% en poids, de préférence au moins 80% en poids, plus préférentiellement au moins 85% en poids, en particulier au moins 90% en poids, plus particulièrement au moins 95% en poids de fer sur base du poids total du matériau **M1;** et moins de 2% en poids de carbone, avantageusement moins de 1,5% en poids, de préférence moins de 1% en poids, plus préférentiellement moins de 0,75% en poids, en particulier moins de 0,5% en poids, plus particulièrement moins de 0,2% en poids, de manière privilégiée moins de 0,1% en poids sur base du poids total du matériau **M1,** plus particulièrement, entre 0,01 et 0,2% en poids de carbone sur base du poids total du matériau **M1** et moins de 2% en poids de molybdène, avantageusement moins de 1,5% en poids, de préférence moins de 1,25% en poids, plus préférentiellement moins de 1% en poids de molybdène sur base du poids total du matériau **M1,** plus particulièrement entre 0,1 et 1% en poids de molybdène sur base du poids total du matériau **M1;** et/ou moins de 5% en poids de chrome, avantageusement moins de 4% en poids, de préférence moins de 3% en poids, plus préférentiellement moins de 2% en poids, en particulier moins de 1% en poids de chrome sur base du poids total du matériau **M1,** plus particulièrement, entre 0,5 et 2% en poids de chrome sur base du poids total du matériau **M1.**

De préférence, le réacteur est alimenté en composé hydrocarbure par des lignes d'alimentation. Le réacteur comprend également des lignes d'effluent ou de sortie permettant d'évacuer le mélange réactionnel du réacteur.

De préférence, les lignes d'alimentation ou de sortie du réacteur sont faites de matériau spécifique capable de résister également à la corrosion, par exemple faites du matériau **M2.** Les lignes d'alimentation peuvent être de forme tubulaire. Alternativement, les lignes d'alimentation ou de sortie peuvent être faites d'un matériau comprenant une couche de base faite d'un matériau **M1** recouverte d'une couche intérieure, en contact avec l'hydrocarbure ou un autre produit de départ, par exemple HF, faite d'un matériau **M2.**

Le réacteur comprend également un ou plusieurs déphlegmateur(s), un ou plusieurs tube(s) plongeur(s), un ou plusieurs dispositif(s) d'introduction des matières premières, une ou plusieurs grille(s) de support et de retenue du catalyseur. Ledit un ou plusieurs déphlegmateur(s) et/ou ledit un ou plusieurs tube(s) plongeur(s) et/ou ledit un ou plusieurs dispositif(s) d'introduction des matières premières et/ou ladite une ou plusieurs grille(s) de support et de retenue du catalyseur peuvent être faites d'un matériau comprenant une couche de base faite d'un matériau **M1** recouverte d'une couche intérieure, en contact avec l'hydrocarbure ou un autre produit de départ, par exemple HF, faite d'un matériau **M2.** Les matériaux **M1** et **M2** sont tels que décrits ci-dessus.

Dans une première réalisation, on augmente la teneur en fluor du composé hydrocarbure en faisant réagir ledit composé avec du fluorure d'hydrogène en présence de ladite composition catalytique, le composé hydrocarbure étant un hydrocarbure halogéné saturé ou un hydrocarbure halogéné insaturé ou un hydrocarbure insaturé. Un composé hydrocarbure est halogéné lorsqu'il comprend au moins un halogène. Un hydrocarbure est insaturé lorsqu'il contient au moins une double liaison carbone-carbone.

Des composés hydrocarbures adéquats comme réactifs de départ pour le procédé de fluoration de cette première réalisation peuvent être des composés hydrocarbures halogénés saturés ou insaturés. Les composés hydrocarbures halogénés saturés incluent ceux de formule générale CₕHₐBr_{b}Cl_{c}F_{d}, où h est un entier entre 1 et 6, a est un entier entre 0 et 13, b est un entier entre 0 et 4, c est un entier entre 0 et 13, d est un entier entre 0 et 13, et la somme de a, b, c et d est égale à 2h+2, pourvu que b+c soit au moins égal à 1. De préférence, les composés hydrocarbures halogénés saturés incluent ceux de formule générale CₕHₐCl_{c}F_{d}, où h est un entier entre 2 to 4, a est un entier entre 0 et 9, c est un entier entre 1 et 9, d est un entier entre 0 et 9, et la somme de a, c et d est égale à 2h+2. Les composés hydrocarbures insaturés halogénés incluent ceux de formule générale CₚHₑBr_{f}Cl_{g}Fₕ, où p est un entier entre 2 et 6, e est un entier entre 0 et 11, f est un entier entre 0 et 2, g est un entier entre 0 et 12, h est un entier entre 0 et 11, pourvu que f + g soit au moins égal à 1, et la somme de e, f, g et h est égale à 2p. De préférence, les composés hydrocarbures insaturés halogénés incluent ceux de formule générale CₚHₑCl_{g}Fₕ, où p est un entier entre 2 et 4, e est un entier entre 0 et 7, g est un entier entre 1 et 8, h est un entier entre 0 et 7, et la somme de e, g et h est égal à 2p. On peut augmenter la teneur en fluor des composés hydrocarbures saturés halogénés de formule CₕHₐBr_{b}Cl_{c}F_{d}, des composés hydrocarbures insaturés halogénés de formule CₚHₑBr_{f}Cl_{g}Fₕ, comme défini plus haut, en faisant réagir lesdits composés hydrocarbures avec HF en phase vapeur en présence de ladite composition catalytique.

Le procédé selon la première réalisation peut être mené dans un réacteur selon la présente invention comprenant un lit catalytique contenant ladite composition catalytique et suivant les conditions opératoires suivantes :
- un rapport molaire HF/composé hydrocarbure entre 1:1 et 150:1, de préférence entre 2:1 et 125:1, plus préférentiellement entre 3:1 et 100:1 ;
- un temps de contact entre 1 et 100 s, de préférence entre 2 et 75 s, en particulier entre 3 et 50 s;
- une pression entre la pression atmosphérique et 20 bara, de préférence entre 2 et 18 bara, plus préférentiellement entre 3 et 15 bara ;
- une température, de préférence du lit catalytique, entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

La quantité de HF mis à réagir avec les composés hydrocarbures doit être au moins stoechiométrique. La quantité stoechiométrique est basée sur le nombre de substituants Br et/ou CI à remplacer par F en plus d'une mole de HF pour saturer la ou les double liaison(s) carbone-carbone s'il y en a.

Des exemples de composés halogénés saturés de formule CₙHₐBr_{b}Cl_{c}F_{d} qui peuvent être mis à réagir avec HF en présence des catalyseurs de cette invention incluent CH₂Cl₂, CH₂Br₂, CHCl₃, CCl₄, C₂Cl₆, C₂BrCl₅, C₂Cl₅F, C₂Cl₄F₂, C₂Cl₃F₃, C₂Cl₂F₄, C₂ClF₅, C₂HCl₅, C₂HCl₄F, C₂HCl₃F₂, C₂HCl₂F₃, C₂HClF₄, C₂HBrF₄, C₂H₂Cl₄, C₂H₂Cl₃F, C₂H₂Cl₂F₂, C₂H₂ClF₃, C₂H₃Cl₃, C₂H₃Cl₂F, C₂H₃ClF₂, C₂H₄Cl₂, C₂H₄ClF, C₃Cl₆F₂, C₃Cl₅F₃, C₃Cl₄F₄, C₃Cl₃F₅, C₃HCl₇, C₃HCl₆F, C₃HCl₅F₂, C₃HCl₄F₃, C₃HCl₃F₄, C₃HCl₂F₅, C₃Cl₂F₆, C₃H₂Cl₆, C₃H₂BrCl₅, C₃H₂Cl₅F, C₃H₂Cl₄F₂, C₃H₂Cl₃F₃, C₃H₂Cl₂F₄, C₃H₂ClF₅, C₃H₃Cl₅, C₃H₃Cl₄F, C₃H₃Cl₃F₂, C₃H₃Cl₂F₃, C₃H₃ClF₄, C₃H₄Cl₄, C₄Cl₄Cl₄, C₄Cl₄Cl₆, C₄H₆Cl₆, C₄H₅Cl₄F, et C₆H₄Cl₈.

Des exemples spécifiques de réactions de fluoration de composés hydrocarbures saturés halogénés qui peuvent être menées à bien dans les conditions décrites ci-dessus en utilisant les catalyseurs de cette invention incluent la conversion du 1,1,2-trichloroéthane (CHCl₂CH₂Cl ou HCC-140) en 1-chloro-2,2-difluoroéthane (CH₂ClCF₂H ou HCFC-142), la conversion du 1,1,1,3,3,3-hexachlorodifluoropropane (CCl₃CF₂CCl₃ ou CFC-212ca) en un mélange de 1,1,3-trichloro-1,2,2,3,3-pentafluoropropane (CCl₂FCF₂CClF₂ ou CFC-215ca) et de 1,3-dichloro-1,1,2,2,3,3-hexafluoropropane (CClF₂CF₂CClF₂ ou CFC-216ca), la conversion du 1,1,1,3,3,3-hexachloropropane (CCl₃CH₂CCl₃ ou HCC-230fa) en 1-chloro-1,1,3,3,3-pentafluoropropane (CF₃CH₂CClF₂ ou HCFC-235fa) et 1,1,1,3,3,3-hexafluoropropane (CF₃CH₂CF₃ ou HFC-236fa), la conversion du 1,1,1,3,3-pentachloropropane (CCl₃CH₂CHCl₂ ou HCC-240fa) en un mélange de 1,1,1,3,3-pentafluoropropane (CHF₂CH₂CF₃ ou HFC-245fa), 1-chloro-3,3,3-trifluoro-1-propène (CHCl=CHCF₃ ou HCFO-1233zd) et 1,3,3,3-tétrafluoropropène (CHF=CHCF₃ ou HFO-1234ze), la conversion du 2,2,3-trichloro-1,1,1,3,3-pentafluoropropane (CF₃CCl₂CClF₂ ou CFC-215aa) en un mélange de 1,1,1,3,3,3-hexachlorodifluoropropane (CF₃CCl₂CF₃ ou CFC-216ca) et 2-chloro-1,1,1,2,3,3,3-heptafluoropropane (CF₃CClFCF₃ ou CFC-217ba), la conversion du 1,1,1,3,3,3-hexachlorodifluoropropane (CF₃CCl₂CF₃ ou CFC-216ca) en 2-chloro-1,1,1,2,3,3,3-heptafluoropropane (CF₃ClFCF₃ ou CFC-217ba), la conversion d'un mélange contenant du 1,1-dichloro-2,2,3,3,3-pentafluoropropane (CF₃CF₂CHCl₂ ou HCFC-225ca) et 1,3-dichloro-1,2,2,3,3-pentafluoropropane (CClF₂CF₂CHClF ou HCFC-225cb) en un mélange de 1-chloro-1,2,2,3,3,3-hexafluoropropane (CF₃CF₂CHClF ou HCFC-226ca) et 1,1,1,2,2,3,3-heptafluoropropane (CF₃CF₂CHF₂ ou HFC-227ca), la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl₂CCl₂CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze), en particulier la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl₂CCl₂CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze), la conversion du 1,1,2-trichloroéthane (CHCl₂CH₂Cl ou HCC-140) en 1-chloro-2,2-difluoroéthane (CH₂ClCF₂H ou HCFC-142).

Des exemples de composés insaturés halogénés ou non de formule CₚHₑBr_{f}Cl_{g}Fₕ et CᵢHᵢ qui peuvent être mis à réagir avec HF en présence des catalyseurs de cette invention incluent C₂Cl₄, C₂BrCl₃, C₂Cl₃F, C₂Cl₂F₂, C₂ClF₃, C₂F₄, C₂HCl₃, C₂HBrCl₂, C₂HCl₂F, C₂HClF₂, C₂HF₃, C₂H₂Cl₂, C₂H₂ClF, C₂H₂F₂, C₂H₃Cl, C₂H₃F, C₂H₄, C₃H₆, C₃H₅Cl, C₃H₄Cl₂, C₃H₃Cl₃, C₃H₂Cl₄, C₃HCl₅, C₃H₂ClF₃, C₃F₃HCl₂, C₃F₂H₂Cl₂, C₃F₄H, ClC₃Cl₆, C₃Cl₅F, C₃Cl₄F₂, C₃Cl₃F₃, C₃Cl₂F₄, C₃ClF₅, C₃HF₅, C₃H₂F₄, C₃F₆, C₄Cl₈, C₄Cl₂F₆, C₄ClF₇, C₄H₂F₆, et C₄HClF₆.

Des exemples spécifiques de réactions de fluoration de composés hydrocarbures insaturés halogénés qui peuvent être menées à bien en utilisant les catalyseurs de cette invention incluent la conversion du 1,2-dichloroéthylène (CHCl=CClH ou HCO-1130) en 1-chloro-2,2-difluoroéthane (CH₂ClCF₂H ou HCFC-142), la conversion du 1,1,2-trichloro-3,3,3-trifluoro-1-propène (CCl₂=CClCF₃ ou CFC-1213xa) en un mélange de 2,3-dichloro-1,1,1,3,3-pentafluoropropane (CF₃CHClCClF₂ ou HCFC-225da), de 2-chloro-1,1,1,3,3,3-hexafluoropropane (CF₃CHClCF₃ ou HCFC-226da) et/ou de 2-chloro-1,1,3,3,3-pentafluoro-1-propène (CF₃CCl=CF₂ ou CFC-1215xc), la conversion de l'hexafluoropropène (CF₃CF=CF₂ ou CFC-1216yc) en 1,1,1,2,3,3,3-heptafluoropropane (CF₃CHFCF₃ ou HFC-227ea), la conversion du 1,1,3,3,3-pentafluoropropène (CF₃CH=CF₂ ou HFO-1225zc) en 1,1,1,3,3,3-hexafluoropropane (CF₃CH₂CF₃ ou HFC-236fa), la conversion du 1,3,3,3-tétrafluoropropène (CF₃CH=CHF ou HFO-1234ze) en 1,1,1,3,3-pentafluoropropane (CF₃CH₂CHF₂ ou HFC-245fa), la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCl₂=CClCH₂Cl ou HCO-1230xa) en 2-chloro-3,3,3-trifluoro-1-propène (CF₃CCl=CH₂ ou HCFO-1233xf) ou 2,3,3,3-tétrafluoropropène (CF₃CF=CH₂ ou HFO-1234yf), la conversion du 2,3,3,3-tétrachloro-1-propène (CCl₃CCl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze), la conversion du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1-chloro-3,3,3-trifluoro-1-propène (CF₃CH=CHCl ou HCFO-1233zd),en particulier la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCl₂=CClCH₂Cl ou HCO-1230xa) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 2,3,3,3-tétrachloro-1-propène (CCl₃Cl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze, la conversion du 1,2-dichloroéthylène (CHCl=CClH ou HCO-1130) en 1-chloro-2,2-difluoroéthane (CH₂ClCF₂H ou HCFC-142).

Préférentiellement, le composé hydrocarbure est choisi dans le groupe constitué du 2-chloro-3,3,3-trifluoro-1-propène (HFCO-1233xf), 1,1,1,2,3-pentachloropropane (HCC-240db), 1,1,2,2,3-pentachloropropane (HCC-240aa), 1,1,2,3-tétrachloro-1-propène (HCO-1230xa), 2,3,3,3-tétrachloro-1-propène (HCO-1230xf), ou leurs mélanges, pour la production du 2,3,3,3-tétrafluoropropène (HFO-1234yf).

Sinon, le composé hydrocarbure est choisi dans le groupe constitué de 1-chloro-3,3,3-trifluoro-1-propène (HCFO-1233zd), 1,1,1,3,3-pentachloropropane (HCC-240fa), 1,1,3,3-tétrachloro-1-propène (HCO-1230za), 1,3,3,3-tétrachloro-1-propène (HCO-1230zd), ou leurs mélanges pour la production du 1,3,3,3-tétrafluoropropène (HFO-1234ze).

Dans une deuxième réalisation, on diminue la teneur en fluor du composé hydrocarbure en déshydrofluorant ledit composé hydrocarbure en présence de ladite composition catalytique, ledit composé hydrocarbure étant un composé hydrocarbure fluoré.

Les composés hydrocarbures fluorés convenant comme matières premières au procédé de déshydrofluoration de cette invention sont typiquement saturés. Les composés hydrocarbures saturés halogénés incluent ceux de formule générale CₙHₐCl_{c}F_{d}, où n est un entier entre 2 et 6, a est un entier entre 1 et 13, c est un entier entre 0 et 12, d est un entier entre 1 et 13, et la somme de a, c et d est égale à 2n+2. De préférence, les composés hydrocarbures saturés halogénés incluent ceux de formule générale CₙHₐCl_{c}F_{d}, où n est un entier entre 2 et 4, a est un entier entre 1 et 9, c est un entier entre 0 et 6, d est un entier entre 1 et 9, et la somme de a, c et d est égale à 2n+2. On peut diminuer la teneur en fluor des composés saturés de formule CₙHₐF_{d} en présence de ladite composition catalytique.

Le procédé selon la deuxième réalisation peut être mené dans un réacteur comprenant un lit catalytique contenant un catalyseur et suivant les conditions opératoires suivantes :
- un rapport molaire HF/composé hydrocarbure entre 1:1 et 150:1, de préférence entre 2:1 et 125:1, plus préférentiellement entre 3:1 et 100:1 ;
- un temps de contact entre 1 et 100 s, de préférence entre 2 et 75 s, en particulier entre 3 et 50 s;
- une pression entre la pression atmosphérique et 20 bara, de préférence entre 2 et 18 bara, plus préférentiellement entre 3 et 15 bara ;
- une température, de préférence du lit catalytique, entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Le produit de la réaction de déshydrofluoration consiste en HF et en le composé hydrocarbure insaturé fluoré résultant de la perte de HF par le réactif initial. Des exemples spécifiques de réactions de déshydrofluoration en phase gazeuse qui peuvent être effectuées en utilisant les catalyseurs de cette invention incluent la conversion du 1,1-difluoroéthane (CHF₂CH₃ ou HFC-152a) en chlorure de vinyle (CHF=CH₂ ou HFO-1141), la conversion du 1,1,1-trifluoroéthane (CF₃CH₃ ou HFC-143a) en fluorure de vinylidène (CF₂=CH₂ ou HFO-1132a), la conversion du 2-chloro-1,1,1-trifluoroéthane (CF₃CH₂Cl ou HCFC-133a) en 2-chloro-1,1-difluoroéthylène (CF₂=CHCl ou HCFO-1122), la conversion du 1,1,1,2-tétrafluoroéthane (CF₃CH₂F ou HFC-134a) en trifluoroéthylène (CF₂=CHF ou HFO-1123), la conversion du 1,1,2,2-tétrafluoroéthane (CHF₂CHF₂ ou HFC-134) en trifluoroéthylène (CF₂=CHF ou HFO-1123), la conversion du 1,1,1,2-tétrafluoropropane (CH₃CHFCF₃ ou HFC-254eb) en 1,1,1-trifluoropropène (CH₂=CHCF₃ ou HFO-1243zf), la conversion du 1,1,1,3,3-pentafluoropropane (CHF₂CH₂CF₃ ou HFC-245fa) en 1,3,3,3-tétrafluoropropène (CHF=CHCF₃ ou HFO-1234ze), la conversion du 1,1,1,2,3,3-hexafluoropropane (CHF₂CHFCF₃ ou HFC-236ea) en 1,2,3,3,3-pentafluoropropène (CHF=CFCF₃ ou HFO-1225ye), la conversion du 1,1,1,3,3,3-hexafluoropropane (CF₃CH₂CF₃ ou HFC-236fa) en 1,1,3,3,3-pentafluoropropène (CF₃CH=CF₂ ou HFO-1225zc), la conversion du 1,1,1,2,3,3-hexafluoropropane (CF₃CF₂CFH₂ ou HFC-236cb) en 1,2,3,3,3-pentafluoropropène (CHF=CFCF₃ ou HFO-1225ye), la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf) et la conversion du 1,1,1,2,3-pentafluoropropane (CF₃CHFCH₂F ou HFC-245eb) en 2,3,3,3-tétrafluoropropène (CF₃CF=CH₂ ou HFO-1234yf).

En particulier, le composé hydrocarbure halogéné est du 1,1,1,2,2-pentafluoropropane (HFC-245cb) pour la production of 2,3,3,3-tétrafluoropropène (HFO-1234yf). Sinon, le composé hydrocarbure halogéné est du 1,1,1,3,3-pentafluoropropane (HFC-245fa) pour la production de 1,3,3,3-tétrafluoropropène (HFO-1234ze).

Dans les procédés selon la première et la deuxième réalisation, la réaction dudit composé hydrocarbure avec du fluorure d'hydrogène peut être effectuée en présence d'oxygène ou de chlore.

Dans une troisième réalisation, on modifie la distribution en fluor dans le composé hydrocarbure en isomérisant ledit composé hydrocarbure en présence de ladite composition catalytique, ledit composé hydrocarbure étant un composé hydrocarbure fluoré.

Dans une quatrième réalisation, on modifie la distribution en fluor dans le composé hydrocarbure en dismutant ledit composé hydrocarbure en phase gazeuse en présence de ladite composition catalytique, ledit composé hydrocarbure étant un composé hydrocarbure chlorofluoré.

Les procédés d'isomérisation et de dismutation des troisième et quatrième réalisations sont menées à bien en phase vapeur en présence de ladite composition catalytique.

Les composés hydrocarbures fluorés adéquats comme matières premières pour les procédés d'isomérisation et de dismutation peuvent être saturés ou insaturés. Les composés hydrocarbures fluorés saturés adéquats pour les procédés d'isomérisation et de dismutation incluent ceux de formule générale CₕHₐBr_{b}Cl_{c}F_{d}, où n est un entier entre 2 et 6, a est un entier entre 0 et 13, b est un entier entre 0 et 4, c est un entier entre 0 et 13, d est un entier entre 1 et 13, et la somme de a, b, c et d est égale à 2n+2, pourvu que a+b+c ≥ 1. Les composés hydrocarbures fluorés insaturés adéquats pour les procédés d'isomérisation et de dismutation incluent ceux de formule générale CₚHₑBr_{f}Cl_{g}Fₕ, où p est un entier entre 2 et 6, e est un entier entre 0 et 11, f est un entier entre 0 et 2, g est un entier entre 0 et 12, h est un entier entre 1 et 11, et la somme de e, f, g et h est égale à 2p, pourvu que la somme e+f+g ≥ 1.

On modifie la distribution de fluor d'un composé hydrocarbure fluoré en réarrangeant les substituants H, Br, CI et F dans la molécule (typiquement un arrangement thermodynamiquement préférentiel) tout en maintenant le même nombre de substituants H, Br, CI et F, respectivement. Dans la présente, ce procédé est appelé isomérisation.

On modifie la distribution de fluor d'un composé hydrocarbure fluoré en échangeant au moins un substituant F de la matière première hydrocarbure halogéné avec au moins un substituant H, Br et/ou CI d'une autre molécule de la matière première hydrocarbure halogéné, de manière à donner la formation d'un ou plusieurs composés hydrocarbures halogénés ayant une teneur en fluor réduite par rapport à la matière première hydrocarbure halogéné et un ou plusieurs composés hydrocarbures halogénés ayant une teneur en fluor accrue par rapport à la matière première hydrocarbure halogéné. Dans la présente, ce procédé est appelé dismutation.

Les réactions d'isomérisation et de dismutation peuvent survenir simultanément.

Que l'on effectue une isomérisation, une dismutation ou à la fois une isomérisation et une dismutation, on peut modifier la distribution en fluor de composés saturés de formule CₙHₐBr_{b}Cl_{c}F_{d} et/ou de composés insaturés de formule CₚHₑBr_{f}Cl_{g}Fₕ en présence d'un catalyseur comme dévoilé plus haut.

Les procédés d'isomérisation et de dismutation sont typiquement menés à bien à des températures entre environ 100°C et 500°C, de préférence entre environ 150°C et environ 400°C. La durée de mise en contact dans le réacteur est typiquement d'environ 1 à environ 120 s, de préférence d'environ 5 à environ 60 s. Les réactions d'isomérisation et de dismutation peuvent être menées à bien en présence d'un gaz inerte, comme l'hélium, l'argon ou l'azote, bien que ce ne soit pas préférentiel. Les réactions d'isomérisation et de dismutation peuvent être menées à bien en présence de HF et HCl.

Préférentiellement, les procédés d'isomérisation peuvent être effectués en utilisant le présent catalyseur et incluent la conversion du 1-chloro-1,1-difluoroéthane (CH₃CF₂Cl ou HCFC-142b) en 1-chloro-2,2-difluoroéthane (CH₂ClCF₂H ou HCFC-142), la conversion du 1,3-dichloro-1,2,2,3,3-pentafluoropropane (CHClFCF₂CF₂Cl ou HCFC-225cb) en 1,1-dichloro-2,2,3,3,3-pentafluoropropane (CHCl₂CF₂CF₃ ou HCFC-225ca), la conversion du 2,2-dichloro-1,1,1,3,3-pentafluoropropane (CHF₂CCl₂CF₃ ou HCFC-225aa) en 1,1-dichloro-2,2,3,3,3-pentafluoropropane (CHCl₂CF₂CF₃ ou HCFC-225ca), la conversion du 1,1,1,2,3-pentafluoropropane (CF₃CHFCH₂F ou HFC-245eb) en 1,1,1,2,2-pentafluoropropane (CF₃CF₂CH₃ ou HFC-245cb), la conversion du 1,1,1,3,3,pentafluoropropane (CHF₂CH₂CF₃ ou HFC-245fa) en 1,1,1,2,3-pentafluoropropane (CF₃CHFCH₂F ou HFC-245eb), la conversion du 1,3,3,3-tétrafluoropropène (CHF=CHCF₃ ou HFO-1234ze) en 2,3,3,3-tétrafluoropropène (CF₃CF=CH₂ ou HFO-1234yf), la conversion du 1,1,3,3-tétrafluoropropène (CF₂=CHCHF₂ ou HFO-1234zc) en 1,3,3,3-tétrafluoropropène (CHF=CHCF₃ ou HFO-1234ze), la conversion du 1-chloro-3,3,3-trifluoro-1-propène (CHCl=CHCF₃ ou HCFO-1233zd) en 2-chloro-3,3,3-trifluoro-1-propène (CH₂=CClCF₃ ou HCFO-1233xf) et la conversion de l'isomère (Z) des hydrochlorofluorooléfines en isomère (E) des hydrochlorofluorooléfines.

En particulier, les isomères (Z) d'hydrochlorofluorooléfines sont les isomères (Z) des hydrochlorofluoropropènes et hydrochlorofluorobutènes. Des exemples spécifiques incluent la conversion du (Z)-1-chloro-3,3,3-trifluoro-1-propène (CHCl=CHCF₃ ou HCFO-1233zd(Z)) en (E)-1-chloro-3,3,3-trifluoro-1-propène (CHCl=CHCF₃ ou HCFO-1233zd(E)), la conversion du (Z)-1,3,3,3-tétrafluoropropène (CHF=CHCF₃ ou HFO-1234ze(Z)) en (E)-1,3,3,3-tétrafluoropropène (CHF=CHCF₃ ou HFO-1234ze(E)), la conversion du (Z)-1,2,3,3,3-pentafluoropropène (CHF=CFCF₃ ou HFO-1225ye(Z)) en (E)-1,2,3,3,3-pentafluoropropène (CHF=CFCF₃ ou HFO-1225ye(E)) et la conversion du (Z)-1,1,1,4,4,4-hexafluoro-2-butène (CF₃CH=CHCF₃ ou HFO-1336mzz(Z)) en (E)-1,1,1,4,4,4-hexafluoro-2-butène (CF₃CH=CHCF₃ ou HFO-1336mzz(E)).

Préférentiellement, les procédés de dismutation peuvent être effectués en utilisant le présent catalyseur et incluent la conversion du chlorofluorométhane (CH₂ClF ou HCFC-31) en difluorométhane (CH₂F₂ ou HFC-32) et dichlorométhane (CH₂Cl₂ ou HCC-30), la conversion du 1-chloro-1,1-difluoroéthane (CH₃CClF₂ ou HCFC-142b) en 1,1,1-trifluoroéthane (CH₃CF₃ ou HFC-143a) et 1,1-dichloro-1-fluoroéthane (CH₃CCl₂F ou HCFC-141b), la conversion du 1-chloro-1,2,2,2-tétrafluoroéthane (CF₃CHClF ou HCFC-124) en pentafluoroéthane (CF₃CHF₂ ou HFC-125) et en 2,2-dichloro-1,1,1-trifluoroéthane (CF₃CHCl₂ ou HCFC-123), la conversion du 1,1,3-trichloro-2,2,3,3-tétrafluoropropane (CHCl₂CF₂CF₂Cl ou HCFC-224ca) en 1,1-dichloro-2,2,3,3,3-pentafluoropropane (CHCl₂CF₂CF₃ ou HCFC-225ca) et 1,1,3,3-tétrachloro-1,2,2-trifluoropropane (CHCl₂CF₂CCl₂F ou HCFC-223ca), la conversion du 1,1,1,3-tétrafluoro-3-chloropropane (CF₃CH₂CHClF ou HCFC-244fa) en 1,1,1,3,3-pentafluoropropane (CHF₂CH₂CF₃ ou HFC-245fa) et en 1,1,1-trifluoro-3,3-dichloropropane (CF₃CH₂CHCl₂ ou HCFC-243fa), la conversion du 1,1,2,3-tétrafluoro-1-chloropropane (CF₂ClCHFCH₂F ou HCFC-244ec) en 1,1,1,2,3-pentafluoropropane (CF₃CHFCH₂F ou HFC-245eb) et en 1,2,3-trifluoro-1,1-dichloropropane (CFCl₂CHFCH₂F ou HCFC-243ed), la conversion du 1,1,2,2-tétrafluoro-1-chloropropane (CF₂ClCF₂CH₃ ou HCFC-244cc) en 1,1,1,2,2-pentafluoropropane (CF₃CF₂CH₃ ou HFC-245cb) et 1,2,2-trifluoro-1,1-dichloropropane (CFCl₂CF₂CH₃ ou HCFC-243cc), la conversion du 3-chloro-2,3,3-trifluoro-1-propène (CH₂=CFCClF₂ ou HCFO-1233yf) en 2,3,3,3-tétrafluoropropène (CF₃CF=CH₂ ou HFO-1234yf) et en 3,3-dichloro-2,3-difluoro-1-propène (CH₂=CFCFCl₂ ou HCFO-1232yf) et la conversion du 3-chloro-1,3,3-trifluoro-1-propène (CHF=CHCClF₂ ou HCFO-1233ze) en 1,3,3,3-tétrafluoropropène (CHF=CHCF₃ ou HFO-1234ze) et 3,3-dichloro-1,3-difluoro-1-propène (CHF=CHCCl₂F ou HCFO-1232ze).

Dans une cinquième réalisation, on diminue la teneur en fluor du composé hydrocarbure en faisant réagir ledit composé hydrocarbure avec du chlorure d'hydrogène en phase gazeuse en présence de ladite composition catalytique, ledit composé hydrocarbure étant un composé hydrocarbure halogéné.

Les composés hydrocarbures fluorés adéquats comme matières premières pour le procédé de cette réalisation peuvent être saturés ou insaturés. Les composés hydrocarbures saturés halogénés adéquats pour les procédés de chlorodéfluoration selon cette invention incluent ceux de formule générale CₙHₐCl_{c}F_{d}, où n est un entier entre 1 et 6, a est un entier entre 0 et 13, c est un entier entre 0 to 13, d est un entier entre 1 to 13, et la somme de a, c et d est égale à 2n+2. Les composés hydrocarbures insaturés halogénés adéquats pour les procédés de chlorodéfluoration selon cette invention incluent ceux de formule générale CₚHₑCl_{g}Fₕ, où p est un entier entre 2 et 6, e est un entier entre 0 et 11, g est un entier entre 0 et 12, h est un entier entre 1 et 11, et la somme de e, g et h est égale à 2p.

Les réactions de chlorodéfluoration sont menées typiquement à des températures d'environ 250°C à 450°C, de préférence d'environ 300°C à environ 400°C. La durée de mise en contact dans le réacteur est typiquement d'environ 1 à environ 120 s. Bien sûr, des durées de contact d'environ 5 à environ 60 s sont possibles. Les réactions sont idéalement menées à pression atmosphérique ou plus.

Les chlorodéfluorations impliquant des hydrocarbures halogénés saturés sont particulièrement dignes d'intérêt. Le rapport molaire de HCl au composé hydrocarbure halogéné saturé se situe typiquement entre environ 1:1 et environ 100:1, de préférence d'environ 3:1 à environ 50:1, et idéalement d'environ 4:1 à environ 30:1. En général, avec une composition catalytique donnée, plus haute est la température, plus longue est la durée de contact, plus grand est le rapport molaire de HCl au composé hydrocarbure halogéné saturé, et plus importante est la conversion des composés à faible teneur en fluor. On peut équilibrer les variables ci-dessus les unes par rapport aux autres pour maximiser la formation de produits chlorés.

Le produit des réactions de chlorodéfluoration comprend typiquement du HCl et du HF non-réagi, de la matière première non-convertie et des composés hydrocarbures saturés halogénés ayant une teneur en fluor plus faible que la matière première par suite de la substitution d'un ou plusieurs substituants fluor par du chlore.

On peut séparer les produits réactionnels obtenus par les procédés détaillés dans l'une quelconque des cinq premières réalisations par des techniques conventionnelles, comme avec des combinaisons incluant, non-limitativement, lavage, décantation ou distillation. Certains des produits des diverses réalisations de cette invention peuvent former un ou plusieurs azéotropes les uns avec les autres ou avec HF.

Les procédés dévoilés dans la présente invention peuvent inclure, en outre, une étape de régénération de ladite composition catalytique en présence d'un flux de régénération comprenant un flux d'air/oxydant. L'oxydant peut être de l'oxygène, de l'air, un mélange d'oxygène et d'azote, du chlore ou un mélange de chlore et d'azote. Quand la régénération est effectuée avec de l'air ou un mélange d'oxygène et d'azote, la proportion d'oxygène peut aller de 5 à 100 % en moles rapporté au mélange d'oxygène et d'azote. L'étape de régénération peut être effectuée en présence d'un flux de régénération contenant (a) de l'oxygène ou de l'air ou un mélange oxygène/azote ou du chlore et (b) HF. Avantageusement, le flux de régénération contiendra au moins 1% en moles d'oxygène rapporté au flux total de régénération. La proportion d'oxygène peut aller de 2 à 98 % en moles rapporté à la quantité totale exprimée en moles d'oxygène et HF, et de 20 à 100 % en moles rapporté à la quantité totale exprimée en moles d'oxygène et d'azote. L'étape de régénération est menée à bien à une température de 250 à 500°C, de préférence de 300 à 450°C, plus préférentiellement de 350 à 400°C. L'étape de régénération peut être menée à bien avec une durée de contact de 1 à 200 s, de préférence de 1 à 150 s, plus préférentiellement de 5 à 100 s, et pour une durée de 1 à 1500 h, de préférence de 2 à 1000 h, plus préférentiellement de 4 à 500 h, idéalement de 10 à 200 h et en particulier de 15 à 150 h. L'étape de régénération peut être menée à bien sous une pression allant de la pression atmosphérique à 20 bara. En particulier, l'étape de régénération peut être menée à bien à une température de 250 à 500°C, avec une durée de contact de 1 à 200 s, pendant 10 à 200 h et sous une pression entre la pression atmosphérique et 20 bara.

Les procédés dévoilés dans la présente invention peuvent comprendre, en outre, l'étape d'activation de ladite composition catalytique en présence d'un flux d'air/oxydant. Avant utilisation, il est préférable que le catalyseur soit soumis à une étape d'activation avec de l'air, de l'oxygène ou du chlore et/ou HF. Par exemple, le catalyseur est préférentiellement soumis à une activation avec de l'air ou de l'oxygène, et HF à une température entre 100 et 500°C, de préférence entre 250 et 500°C et en particulier entre 300 et 400°C. La durée d'activation est préférentiellement de 1 à 200 h et en particulier de 1 à 50 h. Cette activation peut être suivie d'une étape finale d'activation de fluoration en présence d'un oxydant, HF et de composés hydrocarbures. Le rapport molaire HF/composé hydrocarbure va de 2 à 40, et le rapport molaire oxydant/composé hydrocarbure va de 0,04 à 25. La température de l'étape finale d'activation de fluoration peut aller de 300 à 400°C, de préférence pour une durée de 6 à 100 h.

Sinon, la présente invention peut aussi fournir un procédé de modification de la distribution du chlore dans un composé hydrocarbure en présence de ladite composition catalytique. On diminue la teneur en chlore du composé hydrocarbure par déshydrochloration dudit composé hydrocarbure en présence de ladite composition catalytique, ledit composé hydrocarbure étant un composé hydrocarbure chloré. Les composés hydrocarbures chlorés adéquats comme matières premières pour le procédé de déshydrochloration sont typiquement saturés. Les composés hydrocarbures chlorés saturés incluent ceux de formule générale CₙHₐCl_{d}, où n est un entier entre 2 et 6, a est un entier entre 1 to 12, d est un entier entre 1 to 13, et la somme de a et d est égale à 2n+2. Le procédé selon ce mode réalisation alternatif peut être mené dans un réacteur comprenant un lit catalytique contenant un catalyseur et suivant les conditions opératoires suivantes :
- durée de contact de 1 à 100 s, de préférence de 2 à 75 s, en particulier de 3 à 50 s ;
- pression entre la pression atmosphérique et 20 bara, de préférence de 2 à 18 bara, plus préférentiellement de 3 à 15 bara ;
- température, de préférence du lit catalytique, de 200 à 450°C, de préférence de 250 à 400°C, plus préférentiellement de 280°C à 380°C.

Le procédé peut être effectué pendant une durée de 10 à 8000 h, de préférence de 50 à 5000 h, plus préférentiellement de 70 à 1000 h.

Le produit de la réaction de déshydrochloration consiste en HCl et le composé hydrocarbure fluoré insaturé résultant de la perte de HCl du réactif initial. Des exemples spécifiques de réactions de déshydrochloration en phase vapeur peuvent être effectuées en utilisant ladite composition catalytique incluent la conversion du 1-chloro-2,2-difluoroéthane (CH₂ClCF₂H or HCFC-142) en 1,1-difluoroéthylène (CH₂=CF₂ ou HFO-1132a), la conversion du 1,1,1,3-tétrafluoro-3-chloropropane (CF₃CH₂CHClF ou HCFC-244fa) en 1,3,3,3-tétrafluoropropène (CHF=CHCF₃ ou HFO-1234ze), la conversion du 1,1,1-trifluoro-3,3-dichloropropane (CF₃CH₂CHCl₂ ou HCFC-243fa) en 1-chloro-3,3,3-trifluoro-1-propène (CHCl=CHCF₃ ou HCFO-1233zd), la conversion du 2,3-dichloro-1,1,1-trifluoropropane (CF₃CHClCH₂Cl ou HCFC-243db) en 2-chloro-3,3,3-trifluoro-1-propène (CF₃CCl=CH₂ ou HFCO-1233xf), la conversion du 2-chloro-1,1,1,2-tétrafluoropropane (CF₃CFClCH₃ ou HCFC-244bb) en 2,3,3,3-tétrafluoropropène (CF₃CF=CH₂ ou HFO-1234yf) et la conversion du 1,1,1,4,4,4-hexafluoro-2-chlorobutane (CF₃CHClCH₂CF₃ ou HFC-346mdf) en 1,1,1,4,4,4-hexafluoro-2-butène (CF₃CH=CHCF₃ ou HFO-1336mzz). Le procédé de modification de la distribution de chlore dans un composé hydrocarbure peut être mené à bien simultanément avec un procédé de modification de la distribution en fluor dans un autre composé hydrocarbure, par exemple en augmentant ou diminuant la teneur en fluor dans ledit autre composé hydrocarbure comme détaillé plus haut en référence à la première et la deuxième réalisation. Par conséquent, la conversion du 2-chloro-1,1,1,2-tétrafluoropropane (CF₃CFClCH₃ ou HCFC-244bb) en 2,3,3,3-tétrafluoropropène (CF₃CF=CH₂ ou HFO-1234yf) peut être réalisée avec la présente composition catalytique simultanément avec la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf).

Dans une sixième réalisation, on augmente la teneur en fluor d'un premier composé hydrocarbure en faisant réagir ledit premier composé hydrocarbure avec du fluorure d'hydrogène en phase gazeuse en présence de ladite composition catalytique, le premier composé hydrocarbure étant un hydrocarbure halogéné saturé ou un hydrocarbure halogéné insaturé ou un hydrocarbure insaturé et on diminue la teneur en fluor d'un deuxième composé hydrocarbure en déshydrofluorant ledit deuxième composé hydrocarbure en présence de ladite composition catalytique, ledit deuxième composé hydrocarbure étant un composé hydrocarbure fluoré. Le premier composé hydrocarbure étant un hydrocarbure halogéné saturé ou un hydrocarbure halogéné insaturé ou un hydrocarbure insaturé est défini ci-dessus en référence à la première réalisation. Le deuxième composé hydrocarbure est défini ci-dessus en référence à la deuxième réalisation. La fluoration du premier composé hydrocarbure et la déshydrofluoration du deuxième composé hydrocarbure sont préférentiellement effectuées simultanément.

Le procédé selon la sixième réalisation peut être mené dans un réacteur comprenant un lit catalytique contenant un catalyseur et suivant les conditions opératoires suivantes :
- un rapport molaire HF/composé hydrocarbure de 1:1 à 150:1, de préférence de 2:1 à 125:1, plus préférentiellement de 3:1 à 100:1 ;
- une durée de contact de 1 à 100 s, de préférence de 2 à 75 s, en particulier de 3 à 50 s ;
- une pression entre la pression atmosphérique et 20 bara, de préférence entre 2 et 18 bara, plus préférentiellement entre 3 et 15 bara ;
- une température (du lit catalytique) entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Les produits de la réaction sont ceux détaillés en référence à la première et la deuxième réalisation. En particulier, la composition catalytique est utile pour la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf) ou la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf) ou la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf) et la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf) ou la conversion du 1,1,1,2,3-pentafluoropropane (CF₃CHFCH₂F ou HFC-245eb) en 2,3,3,3-tétrafluoropropène (CF₃CF=CH₂ ou HFO-1234yf). En particulier, la composition catalytique est utile pour la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf) et la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf).

Le procédé selon la sixième réalisation peut être mené dans un réacteur comprenant un lit catalytique contenant un catalyseur et suivant les conditions opératoires suivantes :
- un rapport molaire HF/composé hydrocarbure de 1:1 à 150:1, de préférence de 2:1 à 125:1, plus préférentiellement de 3:1 à 100:1 ;
- une durée de contact de 1 à 100 s, de préférence de 2 à 75 s, en particulier de 3 à 50 s ;
- une pression entre la pression atmosphérique et 20 bara, de préférence entre 2 et 18 bara, plus préférentiellement entre 3 et 15 bara ;
- une température, de préférence du lit catalytique, entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

### Exemples

### Exemple 1

Dans un réacteur, on dispose un coupon constitué (i) d'une couche faite d'un matériau **M1** comprenant plus de 95% de fer, moins de 1% de carbone, moins de 1% de molybdène et moins de 1% de chrome et constitué (ii) d'une couche faite d'un matériau **M2** comprenant plus de 40% de nickel et de moins 25% de molybdène et moins de 25% de chrome. Le réacteur comprend également un lit catalytique fixe comprenant un catalyseur d'oxyfluorure de chrome. Le 1,1,1,2,3-pentachloropropane et de l'acide fluorhydrique sont introduits dans le réacteur. La température du lit catalytique varie de 350°C à 400°C. La couche faite du matériau **M1** et la couche faite du matériau **M2** sont disposées l'une contre l'autre par placage par explosion. Au bout de 500h, aucune corrosion n'est observée sur le coupon.

### Exemple 2

L'exemple 1 est reproduit à partir d'un coupon présentant les mêmes caractéristiques que celui de l'exemple 1 mais dont la couche faite du matériau **M1** et la couche faite du matériau **M2** sont disposées l'une contre l'autre par placage par laminage à chaud. Les matériaux **M1** et **M2** sont identiques à ceux de l'exemple 1. Au bout de 500 h, aucune corrosion n'est observée sur le coupon de l'exemple 2.

### Exemple comparatif 1

L'exemple 1 est reproduit à partir d'un coupon présentant les mêmes caractéristiques que celui de l'exemple 1 mais dont la couche faite du matériau **M1** et la couche faite du matériau **M2** sont disposées l'une contre l'autre par soudure sans placage. Les matériaux **M1** et **M2** sont identiques à ceux de l'exemple 1. Au bout de 500 h, un changement de coloration est observé à l'interface entre la couche faite du matériau **M1** et la couche faite du matériau **M2.** Le taux de corrosion du matériau **M2** est de 2,0 mm/an dans ces conditions.

## Revendications

1. Procédé de modification de la distribution en fluor dans un composé hydrocarbure, comprenant une étape de mise en contact entre ledit composé hydrocarbure et une composition catalytique comprenant un catalyseur à base de chrome, ledit procédé étant mis en oeuvre dans un réacteur fait d'un matériau comprenant une couche de base faite d'un matériau **M1** et une couche intérieure faite d'un matériau **M2,** ladite couche de base et ladite couche intérieure étant disposées l'une contre l'autre par placage, **caractérisé en ce que** le placage est réalisé par placage par soudure, placage par explosion, placage par laminage à chaud ou placage par laminage à froid, de préférence par placage par explosion ou placage par laminage à chaud ; le matériau **M2** comprend au moins 40% en poids de nickel sur base du poids total du matériau **M2** et le matériau **M1** comprend au moins 70% de fer sur base du poids total du matériau **M1.**

2. Procédé selon la revendication précédente **caractérisé en ce que** le taux de corrosion du matériau **M2,** mesuré suivant ASTM D 2 328-65 T, est inférieur à 1 mm/an.

3. Procédé selon l'une quelconque des revendications précédentes **caractérisé en ce que** le matériau **M2** est en contact avec le composé hydrocarbure et a une résistance à la traction inférieure à celle du matériau **M1.**

4. Procédé selon l'une quelconque des revendications précédentes **caractérisé en ce que** le matériau **M2** est en contact avec le composé hydrocarbure et a une élongation supérieure à celle du matériau **M1.**

5. Procédé selon l'une quelconque des revendications précédentes **caractérisé en ce que** ladite couche intérieure a une épaisseur comprise entre 0,05 et 10 mm, ladite épaisseur de ladite couche intérieure étant inférieure à celle de ladite couche de base.

6. Procédé selon l'une quelconque des revendications précédentes **caractérisé en ce que** le matériau **M2** comprend au moins 45% en poids de nickel sur base du poids total du matériau **M2.**

7. Procédé selon l'une quelconque des revendications précédentes **caractérisé en ce que** ladite couche de base comprend moins de 0,2% de carbone et/ou moins de 1% de molybdène et/ou moins de 2% de chrome sur base du poids total du matériau **M1.**

8. Procédé selon l'une quelconque des revendications précédentes dans lequel ledit composé hydrocarbure est de formule (I) CX(Y)₂-CX(Y)ₘ-CHₘXY où X et Y représentent indépendamment H, F ou CI et m = 0 ou 1 avec au moins un parmi X ou Y qui est CI ou F.

9. Procédé selon la revendication précédente dans lequel le composé hydrocarbure est choisi dans le groupe constitué du tétrachloropropène, chlorotrifluoropropène, pentachloropropane, dichlorotrifluoropropane, trichlorodifluoropropane, tétrafluorochloropropane, tétrachlorofluoropropane, dichlorodifluoropropène, trichlorofluoropropène, pentafluoropropane et leurs mélanges ; de préférence, le composé hydrocarbure est choisi dans le groupe constitué du 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

10. Procédé selon l'une quelconque des revendications 1 à 7, **caractérisé en ce que** la teneur en fluor du composé hydrocarbure est augmentée en faisant réagir ledit composé hydrocarbure avec du fluorure d'hydrogène en phase gazeuse en présence de ladite composition catalytique, le composé hydrocarbure étant un hydrocarbure saturé halogéné ou un hydrocarbure insaturé halogéné ou un hydrocarbure insaturé.

11. Procédé selon l'une quelconque des revendications 1 à 7, **caractérisé en ce que** la teneur en fluor du composé hydrocarbure est diminuée par déshydrofluoration dudit composé hydrocarbure en présence de ladite composition catalytique, ledit composé hydrocarbure étant un composé hydrocarbure fluoré.

12. Procédé selon l'une quelconque des revendications 1 à 7, **caractérisé en ce que** la distribution en fluor du composé hydrocarbure est modifiée en isomérisant ledit composé hydrocarbure en présence de ladite composition catalytique, ledit composé hydrocarbure étant un composé hydrocarbure fluoré.

13. Procédé selon l'une quelconque des revendications 1 à 7, **caractérisé en ce que** la distribution en fluor du composé hydrocarbure est modifiée en dismutant ledit composé hydrocarbure en phase gazeuse en présence de ladite composition catalytique, ledit composé hydrocarbure étant un composé hydrocarbure chlorofluoré.

14. Procédé selon l'une quelconque des revendications 1 à 7, **caractérisé en ce que** la teneur en fluor du composé hydrocarbure est diminuée en faisant réagir ledit composé hydrocarbure avec du chlorure d'hydrogène en phase gazeuse en présence de ladite composition catalytique, ledit composé hydrocarbure étant un composé hydrocarbure halogéné contenant au moins un atome de fluor.

15. Procédé selon l'une quelconque des revendications 1 à 7, **caractérisé en ce que** la teneur en fluor d'un premier composé hydrocarbure est augmentée en faisant réagir ledit premier composé hydrocarbure avec du fluorure d'hydrogène en phase gazeuse en présence d'une composition catalytique comprenant un catalyseur à base de chrome, le premier composé hydrocarbure étant un hydrocarbure saturé halogéné ou un hydrocarbure insaturé halogéné ou un hydrocarbure insaturé, et **en ce que** la teneur en fluor d'un second composé hydrocarbure est diminuée en déshydrofluorant ledit second composé hydrocarbure en présence de ladite composition catalytique, ledit second composé hydrocarbure étant un composé hydrocarbure fluoré.

## Patentansprüche

1. Verfahren zur Modifikation der Verteilung von Fluor in einer Kohlenwasserstoffverbindung, umfassend einen Schritt des Inkontaktbringens der Kohlenwasserstoffverbindung mit einer katalytischen Zusammensetzung, die einen Katalysator auf der Basis von Chrom umfasst, wobei das Verfahren in einem Reaktor durchgeführt wird, der aus einem Material hergestellt ist, das eine aus einem Material **M1** hergestellte Grundschicht und eine aus einem Material **M2** hergestellte Innenschicht umfasst, wobei die Grundschicht und die Innenschicht durch Plattieren aneinander anliegend angeordnet sind, **dadurch gekennzeichnet, dass** das Plattieren mittels Schweißplattieren, Sprengplattieren, Warmwalzplattieren oder Kaltwalzplattieren, vorzugsweise mittels Sprengplattieren oder Warmwalzplattieren erfolgt, wobei das Material **M2** mindestens 40 Gew.-% Nickel bezogen auf das Gesamtgewicht des Materials **M2** umfasst und das Material **M1** mindestens 70 Gew.-% Eisen bezogen auf das Gesamtgewicht des Materials **M1** umfasst.

2. Verfahren nach dem vorhergehenden Anspruch, **dadurch gekennzeichnet, dass** die gemäß ASTM D 2 328-65 T gemessene Korrosionsrate des Materials **M2** weniger als 1 mm/Jahr beträgt.

3. Verfahren nach einem der vorhergehenden Ansprüche, **dadurch gekennzeichnet, dass** das Material **M2** mit der Kohlenwasserstoffverbindung in Kontakt ist und eine niedrigere Zugfestigkeit als das Material **M1** hat.

4. Verfahren nach einem der vorhergehenden Ansprüche, **dadurch gekennzeichnet, dass** das Material **M2** mit der Kohlenwasserstoffverbindung in Kontakt ist und eine größere Streckung als das Material **M1** aufweist.

5. Verfahren nach einem der vorhergehenden Ansprüche, **dadurch gekennzeichnet, dass** die Innenschicht eine Dicke zwischen 0,05 und 10 mm aufweist, wobei die Dicke der Innenschicht kleiner als diejenige der Grundschicht ist.

6. Verfahren nach einem der vorhergehenden Ansprüche, **dadurch gekennzeichnet, dass** das Material **M2** mindestens 45 Gew.-% Nickel bezogen auf das Gesamtgewicht des Materials **M2** umfasst.

7. Verfahren nach einem der vorhergehenden Ansprüche, **dadurch gekennzeichnet, dass** die Grundschicht weniger als 0,2 % Kohlenstoff und/oder weniger als 1 % Molybdän und/oder weniger als 2 % Chrom bezogen auf das Gesamtgewicht des Materials **M1** aufweist.

8. Verfahren nach einem der vorhergehenden Ansprüche, wobei die Kohlenwasserstoffverbindung die Formel (I) CX(Y)₂-CX(Y)ₘ-CHₘXY aufweist, worin X und Y unabhängig H, F oder Cl darstellen und m = 0 oder 1, wobei mindestens eines aus X oder Y Cl oder F ist.

9. Verfahren nach dem vorhergehenden Anspruch, wobei die Kohlenwasserstoffverbindung aus der Gruppe ausgewählt ist, die aus Tetrachlorpropen, Chlortrifluorpropen, Pentachlorpropan, Dichlortrifluorpropan, Trichlordifluorpropan, Tetrafluorchlorpropan, Tetrachlorfluorpropan, Dichlordifluorpropen, Trichlorfluorpropen, Pentafluorpropan und deren Gemischen besteht, vorzugsweise die Kohlenwasserstoffverbindung aus der Gruppe ausgewählt ist, die aus 2-Chlor-3,3,3-trifluor-1-propen (HFCO-1233xf), 2,3-Dichlor-1,1,1-trifluorpropan (HCFC-243db), 1,1,1,2,3-Pentachlorpropan (HCC-240db), 1,1,2,2,3-Pentachlorpropan (HCC-240aa), 1,1,1,3,3-Pentachlorpropan (HCC-240fa), 1,1,2,3-Tetrachlor-1-propen (HCO-1230xa), 2,3,3,3-Tetrachlor-1-propen (HCO-1230xf), 1,1,3,3-Tetrachlor-1-propen (HCO-1230za), 1,3,3,3-Tetrachlor-1-propen (HCO-1230zd), 1,1,1,2,2-Pentafluorpropan (HFC-245cb) und 1-Chlor-3,3,3-trifluor-1-propen (HCFO-1233zd) besteht.

10. Verfahren nach einem der Ansprüche 1 bis 7, **dadurch gekennzeichnet, dass** der Fluorgehalt der Kohlenwasserstoffverbindung erhöht wird, indem man die Kohlenwasserstoffverbindung mit Fluorwasserstoff in der Gasphase in Gegenwart der katalytischen Zusammensetzung reagieren lässt, wobei die Kohlenwasserstoffverbindung ein gesättigter Halogenkohlenwasserstoff oder ein ungesättigter Halogenkohlenwasserstoff oder ein ungesättigter Kohlenwasserstoff ist.

11. Verfahren nach einem der Ansprüche 1 bis 7, **dadurch gekennzeichnet, dass** der Fluorgehalt der Kohlenwasserstoffverbindung durch Dehydrofluorierung der Kohlenwasserstoffverbindung in Gegenwart der katalytischen Zusammensetzung verringert wird, wobei die Kohlenwasserstoffverbindung eine Fluorkohlenwasserstoffverbindung ist.

12. Verfahren nach einem der Ansprüche 1 bis 7, **dadurch gekennzeichnet, dass** die Fluorverteilung der Kohlenwasserstoffverbindung modifiziert wird, indem man die Kohlenwasserstoffverbindung in Gegenwart der katalytischen Zusammensetzung isomerisiert, wobei die Kohlenwasserstoffverbindung eine Fluorkohlenwasserstoffverbindung ist.

13. Verfahren nach einem der Ansprüche 1 bis 7, **dadurch gekennzeichnet, dass** die Fluorverteilung der Kohlenwasserstoffverbindung modifiziert wird, indem man die Kohlenwasserstoffverbindung in der Gasphase in Gegenwart der katalytischen Zusammensetzung dismutiert, wobei die Kohlenwasserstoffverbindung eine Chlorfluorkohlenwasserstoffverbindung ist.

14. Verfahren nach einem der Ansprüche 1 bis 7, **dadurch gekennzeichnet, dass** der Fluorgehalt der Kohlenwasserstoffverbindung verringert wird, indem man die Kohlenwasserstoffverbindung mit Chlorwasserstoff in der Gasphase in Gegenwart der katalytischen Zusammensetzung umsetzt, wobei die Kohlenwasserstoffverbindung eine Halogenkohlenwasserstoffverbindung ist, die mindestens ein Fluoratom enthält.

15. Verfahren nach einem der Ansprüche 1 bis 7, **dadurch gekennzeichnet, dass** der Fluorgehalt einer ersten Kohlenwasserstoffverbindung erhöht wird, indem man die erste Kohlenwasserstoffverbindung mit Fluorwasserstoff in der Gasphase in Gegenwart einer katalytischen Zusammensetzung umsetzt, die einen Katalysator auf der Basis von Chrom umfasst, wobei die erste Kohlenwasserstoffverbindung ein gesättigter Halogenkohlenwasserstoff oder ein ungesättigter Halogenkohlenwasserstoff oder ein ungesättigter Kohlenwasserstoff ist, und dadurch, dass der Fluorgehalt einer zweiten Kohlenwasserstoffverbindung durch Dehydrofluorierung der zweiten Kohlenwasserstoffverbindung in Gegenwart der katalytischen Zusammensetzung verringert wird, wobei die zweite Kohlenwasserstoffverbindung eine Fluorkohlenwasserstoffverbindung ist.

## Claims

1. Process for modifying the fluorine distribution in a hydrocarbon compound, comprising a step of placing in contact between said hydrocarbon compound and a catalytic composition comprising a chromium-based catalyst, said process being performed in a reactor made of a material comprising a base layer made of a material **M1** and an inner layer made of a material **M2,** said base layer and said inner layer being laid against each other by plating, **characterized in that** the plating is performed by solder plating, explosion plating, hot roll plating or cold roll plating, preferably by explosion plating or hot roll plating; the material **M2** comprises at least 40% by weight of nickel on the basis of the total weight of the material **M2,** and the material **M1** comprises at least 70% of iron on the basis of the total weight of the material **M1.**

2. Process according to the preceding claim, **characterized in that** the degree of corrosion of the material **M2,** measured according to ASTM D 2 328-65 T, is less than 1 mm/year.

3. Process according to either of the preceding claims, **characterized in that** the material **M2** is in contact with the hydrocarbon compound and has a tensile strength less than that of the material **M1.**

4. Process according to any one of the preceding claims, **characterized in that** the material **M2** is in contact with the hydrocarbon compound and has an elongation greater than that of the material **M1.**

5. Process according to any one of the preceding claims, **characterized in that** said inner layer has a thickness of between 0.05 and 10 mm, said thickness of said inner layer being less than that of said base layer.

6. Process according to any one of the preceding claims, **characterized in that** the material **M2** comprises at least 45% by weight of nickel on the basis of the total weight of the material **M2.**

7. Process according to any one of the preceding claims, **characterized in that** said base layer comprises less than 0.2% of carbon and/or less than 1% of molybdenum and/or less than 2% of chromium on the basis of the total weight of the material **M1.**

8. Process according to any one of the preceding claims, in which said hydrocarbon compound is of formula (I) CX(Y)₂-CX(Y)ₘ-CHₘXY, in which X and Y independently represent H, F or Cl and m = 0 or 1 with at least one from among X or Y which is Cl or F.

9. Process according to the preceding claim, in which the hydrocarbon compound is chosen from the group consisting of tetrachloropropene, chlorotrifluoropropene, pentachloropropane, dichlorotrifluoropropane, trichlorodifluoropropane, tetrafluorochloropropane, tetrachlorofluoropropane, dichlorodifluoropropene, trichlorofluoropropene, pentafluoropropane and mixtures thereof; preferably, the hydrocarbon compound is chosen from the group consisting of 2-chloro-3,3,3-trifluoro-1-propene (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tetrachloro-1-propene (HCO-1230xa), 2,3,3,3-tetrachloro-1-propene (HCO-1230xf), 1,1,3,3-tetrachloro-1-propene (HCO-1230za), 1,3,3,3-tetrachloro-1-propene (HCO-1230zd), 1,1,1,2,2-pentafluoropropane (HFC-245cb) and 1-chloro-3,3,3-trifluoro-1-propene (HCFO-1233zd).

10. Process according to any one of Claims 1 to 7, **characterized in that** the fluorine content of the hydrocarbon compound is increased by reacting said hydrocarbon compound with hydrogen fluoride in the gas phase in the presence of said catalytic composition, the hydrocarbon compound being a saturated halogenated hydrocarbon or an unsaturated halogenated hydrocarbon or an unsaturated hydrocarbon.

11. Process according to any one of Claims 1 to 7, **characterized in that** the fluorine content of the hydrocarbon compound is reduced by dehydrofluorination of said hydrocarbon compound in the presence of said catalytic composition, said hydrocarbon compound being a fluorinated hydrocarbon compound.

12. Process according to any one of Claims 1 to 7, **characterized in that** the fluorine distribution of the hydrocarbon compound is modified by isomerizing said hydrocarbon compound in the presence of said catalytic composition, said hydrocarbon compound being a fluorinated hydrocarbon compound.

13. Process according to any one of Claims 1 to 7, **characterized in that** the fluorine distribution of the hydrocarbon compound is modified by dismutating said hydrocarbon compound in the gas phase in the presence of said catalytic composition, said hydrocarbon compound being a chlorofluorinated hydrocarbon compound.

14. Process according to any one of Claims 1 to 7, **characterized in that** the fluorine content of the hydrocarbon compound is reduced by reacting said hydrocarbon compound with hydrogen chloride in the gas phase in the presence of said catalytic composition, said hydrocarbon compound being a halogenated hydrocarbon compound containing at least one fluorine atom.

15. Process according to any one of Claims 1 to 7, **characterized in that** the fluorine content of a first hydrocarbon compound is increased by reacting said first hydrocarbon compound with hydrogen fluoride in the gas phase in the presence of a catalytic composition comprising a chromium-based catalyst, the first hydrocarbon compound being a saturated halogenated hydrocarbon or an unsaturated halogenated hydrocarbon or an unsaturated hydrocarbon, and **in that** the fluorine content of a second hydrocarbon compound is reduced by dehydrofluorinating said second hydrocarbon compound in the presence of said catalytic composition, said second hydrocarbon compound being a fluorinated hydrocarbon compound.
